(19) Europäisches Patentamt
European Patent Office
Office européen des brevets

(11) **EP 3 195 925 B1**

(12) **EUROPÄISCHE PATENTSCHRIFT**

(45) Veröffentlichungstag und Bekanntmachung des Hinweises auf die Patenterteilung:
**04.05.2022 Patentblatt 2022/18**

(21) Anmeldenummer: **16000563.3**

(22) Anmeldetag: **08.03.2016**

(51) Internationale Patentklassifikation (IPC):
**B01F 15/00** *(2006.01)* **F17C 13/00** *(2006.01)*

(52) Gemeinsame Patentklassifikation (CPC):
**F17C 13/002; G05D 11/03**

(54) **GASVERDÜNNUNGSSYSTEM**

GAS DILUTION SYSTEM

SYSTEME DE DILUTION DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.01.2016 DE 102016000518**

(43) Veröffentlichungstag der Anmeldung:
**26.07.2017 Patentblatt 2017/30**

(73) Patentinhaber: **Linde GmbH**
**82049 Pullach (DE)**

(72) Erfinder: **Adam, Peter**
**81369 München (DE)**

(74) Vertreter: **Lu, Jing et al**
**Linde GmbH**
**Intellectual Property EMEA**
**Dr.-Carl-von-Linde-Straße 6-14**
**82049 Pullach (DE)**

(56) Entgegenhaltungen:
EP-A1- 0 503 996 EP-A1- 0 541 234
EP-A1- 0 837 280 EP-A2- 1 515 080
WO-A1-2007/050400 DE-A1- 1 941 669
US-A- 5 297 432 US-A- 5 671 767

**Beschreibung**

Die Erfindung betrifft ein Gasverdünnungssystem wenigstens zwei Gasdurchflusssteuerelementen und wenigstens zwei Gasbehälter für ein Gasverdünnungssystem.

Stand der Technik

Im Zuge einer Gasverdünnung können unterschiedliche Gase miteinander vermischt werden, und ein verdünntes Gasgemisch kann erzeugt werden. Der Volumenanteil der einzelnen Gase in diesem verdünnten Gasgemisch wird bei der Gasverdünnung in einem gewünschten Verdünnungsverhältnis eingestellt. Beispielsweise kann ein derartiges verdünntes Gasgemisch erzeugt und zur Kalibrierung von Gassensoren bzw. eines Gasanalysenmessgeräts verwendet werden.

Entsprechende Gasverdünnungssysteme sind häufig sehr sperrig und platzintensiv konstruiert. Zumeist weisen derartige Gasverdünnungssysteme eine Vielzahl von einzelnen Bauteilen auf, beispielsweise eine Vielzahl von einzelnen Messblenden, Ventilen und Druckreglern. Gasverdünnungssysteme sind daher oftmals sehr kostenintensiv in Betrieb und Herstellung und kompliziert zu handhaben. Beispiele hierfür sind die DE194166, weiche eine Vorrichtung zum Mischen von Gasen beschreibt und bei denen mehrere Einzelkomponenten verbaut sind und insbesondere eine spezielle Mischkammer beschreibt, oder die WO2007/050400A1 in der die Mischung von medizinischer Luft aus Sauerstoff und Stickstoff für ein Krankenhaus beschrieben wird.

Die EP0503996A1 beschreibt ein Verdünnungssystem mit kalibrierten Messblenden unter Verwendung von kritischen Strömungsbedingungen.

Es ist wünschenswert, ein verbessertes und vereinfachtes Gasverdünnungssystem bereitzustellen.

Offenbarung der Erfindung

Erfindungsgemäß ein Gasverdünnungssystem wenigstens zwei Gasdurchflusssteuerelementen und wenigstens zwei Gasbehältern für ein Gasverdünnungssystem mit den Merkmalen des Anspruch 1 vorgeschlagen. Vorteilhafte Ausgestaltungen sind Gegenstand der Unteransprüche sowie der nachfolgenden Beschreibung.

Das Gasverdünnungssystem weist wenigstens zwei Gasdurchflusssteuerelemente auf. Jedes dieser Gasdurchflusssteuerelemente weist jeweils eine integrierte Messblende auf, ist als Aufsatz nach Art eines Ventils ausgebildet und ist jeweils mit einem Gasbehälter verbunden.

Das Gasverdünnungssystem ist dazu eingerichtet, über die wenigstens zwei Gasdurchflusssteuerelemente jeweils Gas mit einem vorgegebenen Durchfluss aus dem jeweiligen verbundenen Gasbehälter zu entnehmen, die entnommenen Gase in einem vorgegebenen Verhältnis, hier als Verdünnungsverhältnis bezeichnet, zu einem verdünnten Gasgemisch zu vermischen und das verdünnte Gasgemisch an einem Ausgang bereitzustellen.

Die Gasdurchflusssteuerelemente sind direkt auf den entsprechenden Gasbehälter aufgesetzt bzw. direkt mit diesem verbunden. Im Gegensatz zu einem herkömmlichen Gasverdünnungssystem ist somit für jeden Gasbehälter ein eigenes Gasdurchflusssteuerelement vorgesehen, welches direkt mit diesem verbunden ist. Die Gasdurchflusssteuerelemente können derart ausgebildet sein, dass sie flexibel mit dem entsprechenden Gasbehälter verbunden auch wieder von diesem gelöst werden können, beispielsweise über eine Schraub- oder Steckverbindung. Gasdurchflusssteuerelemente und Gasbehälter können insbesondere auch fest bzw. untrennbar miteinander verbunden sein.

Die integrierten Messblenden, im Englischen auch als "orifice" bezeichnet, können beispielsweise als kalibrierte, integrierte Durchflussregler ausgebildet sein. Blendendurchmesser der einzelnen Messblenden kann variiert werden. Und dadurch kann über die integrierten Messblenden der Durchfluss eingestellt werden, mit welchem Gas aus dem jeweiligen Gasbehälter entnommen wird.

Durch die Gasdurchflusssteuerelemente kann auf einfache Weise eine zur Erzeugung des verdünnten Gasgemischs gewünschte Menge des jeweiligen Gases aus dem Gasbehälter entnommen werden. So kann das verdünnte Gasgemisch mit dem gewünschten Verdünnungsverhältnis nur durch Einstellen des Gasdurchflusses über die Gasdurchflusssteuerelemente bzw. die Messblenden erzeugt werden.

Es sei darauf hingewiesen, dass über die jeweiligen Gasdurchflusssteuerelemente je nach Bedarf zweckmäßigerweise einzelne Gase (z.B. Stickstoff, synthetische Luft) oder auch eine Mischung aus mehreren einzelnen Gasen (beispielsweise eine Mischung einer speziellen Menge von $CO_2$ in einer speziellen Menge von Stickstoff) entnommen werden können.

Durch das Gasverdünnungssystem, insbesondere durch die Verwendung der Gasdurchflusssteuerelemente, kann auf einfache Weise das verdünnte Gasgemisch in dem gewünschten Verdünnungsverhältnis erzeugt werden. Das Gasverdünnungssystem kann kompakt und platzsparend konstruiert werden und kann preisgünstig, aufwandswarm und unkompliziert hergestellt und betrieben werden. Durch die kompakte Bauweise ist das Gasverdünnungssystem insbesondere auch für mobile Anwendungen geeignet. Insbesondere kann das Gasverdünnungssystem ohne elektrischen Strom betrieben werden. Zweckmäßigerweise sind sämtliche zur Regelung bzw. zum Einstellen des gewünschten Verdünnungsverhältnisses benötigten Elemente in die Gasdurchflusssteuerelemente integriert.

Insbesondere kann ein Verdünnungsverhältnis im ppb-Bereich erzeugt werden, was einem Volumenmischungsverhältnis von insbesondere $10^{-7}$ Vol.-% ent-

spricht. Das verdünnte Gasgemisch lässt sich durch das Gasverdünnungssystem mit einer relativen Messunsicherheit von insbesondere weniger als 1% erzeugen. Beispielsweise kann das Verdünnungsverhältnis mit Durchflüssen von 0,25 l/min bis 8,00 l/min in Schritten von insbesondere 1/33 verändert werden.

Mittels des Gasverdünnungssystems kann insbesondere auch ein verdünntes Gasgemisch mit reaktiven Gasen als Komponenten erzeugt werden. Beispielsweise können reaktive Gase wie $H_2S$, $SO_2$, $NO$, $NH_3$ verwendet werden. Mit herkömmlichen Gasverdünnungssystemen ist es zumeist nicht oder nur mit großem Aufwand möglich, derartige Gasgemische herzustellen, da derartige reaktive Gase nur eine begrenzte Stabilität in Hochdruck-Gasbehältern besitzen, insbesondere im ppb-Bereich und im niedrigen ppm-Bereich. Insbesondere besteht dabei die Gefahr, dass die reaktiven Gase durch Adsorptionseffekte mit der Behälterwand des Gasbehälters reagieren. Durch das erfindungsgemäße Gasverdünnungssystem können diese Nachteile jedoch überwunden werden. Weiterhin wird es durch die Erfindung ermöglicht, verdünnte Gasgemische dynamisch herzustellen, wohingegen mit herkömmlichen Mitteln oftmals nur eine statische Herstellung von verdünnten Gasgemischen möglich ist.

Über den Ausgang kann das Gasverdünnungssystem mit einem Gasverbraucher verbunden werden. Beispielsweise kann das verdünnte Gasgemisch zur Kalibrierung von Gassensoren bzw. eines Gasanalysenmessgeräts verwendet werden. Ein entsprechender Gassensor bzw. ein entsprechendes Gasanalysenmessgerät kann zu diesem Zweck über den Ausgang mit dem Gasverdünnungssystem verbunden werden.

Das vorgegebene Verdünnungsverhältnis des verdünnten Gasgemischs über die integrierten Messblenden der wenigstens zwei Gasdurchflusssteuerelemente ist einstellbar. Sämtliche zum Einstellen des gewünschten Verdünnungsverhältnisses benötigten Elemente sind somit insbesondere in die Gasdurchflusssteuerelemente integriert. Zur Regelung des Verdünnungsverhältnisses werden insbesondere keine zusätzlichen Elemente benötigt.

Das vorgegebene Verdünnungsverhältnis über Blendendurchmesser der integrierten Messblenden der wenigstens zwei Gasdurchflusssteuerelemente ist einstellbar. Zweckmäßigerweise kann der Durchfluss des jeweiligen Gases durch das jeweilige Gasdurchflusssteuerelement über den entsprechenden Blendendurchmesser eingestellt werden. Das gewünschte Verdünnungsverhältnisses kann insbesondere erzeugt werden, indem die Durchflüsse der einzelnen Gase eingestellt werden, zweckmäßigerweise über die einzelnen Blendendurchmesser.

Vorteilhafterweise umfassen die wenigstens zwei Gasdurchflusssteuerelemente jeweils weiterhin einen integrierten Gasdruckregler. Insbesondere lässt sich über den integrierten Gasdruckregler sowie die integrierte Messblende der Durchfluss des jeweiligen Gases und somit insbesondere das Verdünnungsverhältnis einstellen. Insbesondere können die Gasdurchflusssteuerelemente in diesem Fall als sogenannte VIPR-Elemente (sog. "Valve with Integrated Pressure Regulator") ausgebildet sein.

Vorzugsweise sind die integrierten Messblenden jeweils als eine integrierte Messblende für kritische Strömungen (sog. "critical orifice") ausgebildet. Mittels einer derartigen Messblende kann insbesondere eine kritische Strömung ("critical flow", "chocked flow") erzeugt werden. Der Durchfluss bzw. die Durchflussmenge einer derartigen kritischen Strömung ist insbesondere unabhängig vom Druck stromabwärts der Messblende und nur abhängig von der Temperatur und dem Druck bzw. der Dichte stromaufwärts der Messblende. Zweckmäßigerweise kann der Durchfluss in diesem Fall mittels des integrierten Gasdruckreglers und durch zweckmäßiges Einstellen des Drucks stromaufwärts der Messblende eingestellt bzw. geregelt werden.

Insbesondere kann das Gasverdünnungssystem in diesem Fall gemäß der Norm ISO 6145-6:2003 ("Gas analysis - Preparation of calibration gas mixtures using dynamic volumetric methods - Part 6: Critical orifices") konstruiert werden und die von dieser Norm gestellten Anforderungen können eingehalten werden.

Gemäß einer vorteilhaften Ausgestaltung können die Gasdurchflusssteuerelemente fest mit dem jeweiligen Gasbehälter verbunden sein. Gasdurchflusssteuerelement und Gasbehälter bilden in diesem Fall vorteilhafterweise eine gemeinsame bauliche Einheit. Das Gasdurchflusssteuerelement ist besonders bevorzugt als Aufsatz des Gasbehälters ausgebildet, in welchem ein Gasdruckregler und eine Messblende integriert sind. Derartige bauliche Einheiten können auf unkomplizierte Weise mit dem übrigen Gasverdünnungssystem verbunden werden. Wird ein Gasbehälter ausgetauscht, beispielsweise wenn dieser leer ist, werden mit einem neuen Gasbehälter automatisch eine neue Messblende und insbesondere ein neuer Gasdruckregler mitgeliefert. Insbesondere können derartige bauliche Einheiten auf einfache Weise wieder befüllt werden. Durch die integriere Messblende und den zweckmäßigerweise integrierten Gasdruckregler sind insbesondere keine zusätzlichen Elemente zum Wiederbefüllen notwendig.

Insbesondere ist es für die Gasverdünnung nicht notwendig, zusätzliche Gasdruckregler oder Messblenden zu verwenden; sämtliche benötigte Elemente sind bereits in die Gasbehälter integriert. Somit besteht nicht die Gefahr, dass einzelne Gasdruckregler oder Messblenden verloren oder verlegt werden können. Bei einem Defekt eines einzelnen Gasdruckreglers oder einer Messblende muss nicht das gesamte Gasverdünnungssystem stillgelegt werden und es müssen keine aufwendigen Reparatur- oder Austauscharbeiten durchgeführt werden, sondern es wird insbesondere lediglich der jeweiligen Gasbehälter ausgetauscht.

Besonders vorteilhafterweise können Gasdurchflusssteuerelement und Gasbehälter als Druckgasbehälter mit integriertem Flaschenventil, integriertem Druckminderer und als integrierter Durchflussregler ausgebildeter Messblende ausgebildet sein, welcher von der Anmelderin insbesondere unter der Bezeichnung ECOCYL® vertrieben wird. Das Gasdurchflusssteuerelement ist bei einem derartigen Druckgasbehälter zweckmäßigerweise vollständig in einem Schutzkäfig des Druckgasbehälters integriert. Derartige Druckgasbehälter stellen ein gebrauchsfertiges System dar, sind sicher und einfach zu handhaben und können auf einfache Weise wieder befüllt werden. Zur Entnahme von Gas kann das jeweilige Flaschenventil geöffnet werden und aus voreingestellten Flussraten kann eine gewünschte Flussrate ausgewählt werden.

Gasdurchflusssteuerelement und Gasbehälter können besonders bevorzugt auch als eine Gasflasche mit integrierten Druckminderer ausgebildet sein, welche von der Anmelderin insbesondere unter der Bezeichnung LIV vertrieben werden. Eine derartige Gasflasche ist ein sehr leichtes und sofort einsetzbares mobiles System für die Versorgung mit Gasen. Durch den integrierten Druckminderer ist eine entsprechende Gasflasche sofort zur Anwendung bereit. Insbesondere umfasst eine derartige Gasflasche einen Aluminiumbehälter und einen entsprechenden integrierten Druckminderer.

Weiterhin weist das Gasverdünnungssystem einen Übermengenregler auf, bevorzugt ein Nadelventil und/oder einen Gegendruckregler ("back pressure regulator"). Mittels des Übermengenreglers kann beispielsweise verdünntes Gas, welches von einem am Ausgang angeschlossenen Gasverbraucher nicht benötigt wird, ausgelassen werden. Insbesondere kann eine von dem Gasverbraucher geforderte Durchflussmenge an verdünntem Gas aufrechterhalten werden.

Vorteilhafterweise weist das Gasverdünnungssystem weiterhin eine Gaszusammenführung auf, welche mit den wenigstens zwei Gasdurchflusssteuerelementen verbunden ist. Durch diese Gaszusammenführung werden die aus den einzelnen Gasbehältern entnommenen Gase zusammengeführt. Beispielsweise kann die Gaszusammenführung eine zweckmäßige Anzahl von Gasleitungen aufweisen.

Besonders bevorzugt ist diese Gaszusammenführung als ein Kreuzstück ("cross fitting") von Gasleitungen bzw. als ein Glasleitungskreuz ausgebildet. Ein derartiges Kreuzstück weist insbesondere vier Anschlüsse auf. Ein erster Anschluss des Kreuzstücks ist bevorzugt mit einer ersten Anzahl der wenigstens zwei Gasdurchflusssteuerelemente verbunden, ein zweiter Anschluss des Kreuzstücks ist vorzugsweise mit einer zweiten Anzahl der wenigstens zwei Gasdurchflusssteuerelemente verbunden. Über den ersten Anschluss werden somit entsprechende Mengen einer entsprechenden ersten Anzahl von Gasen entnommen und über den zweiten Anschluss werden entsprechende Mengen einer entsprechenden zweiten Anzahl von Gasen entnommen und in dem Kreuzstück vermischt. In dem Kreuzstück wird somit das verdünnte Gasgemisch erzeugt. Vorzugsweise ist ein dritter Anschluss des Kreuzstücks mit dem Ausgang verbunden, an welchem das verdünnte Gasgemisch bereitgestellt wird. Mit einem vierten Anschluss des Kreuzstücks ist vorteilhafterweise der Übermengenregler verbunden.

Besonders bevorzugt ist der erste Anschluss des Kreuzstücks mit genau einem Gasdurchflusssteuerelement verbunden. Dieses Gasdurchflusssteuerelement ist zweckmäßigerweise für einen Gasbehälter eines Ausgleichsgases, z.B. Stickstoff, vorgesehen. Der zweite Anschluss des Kreuzstücks ist in diesem Fall vorteilhafterweise mit den restlichen Gasdurchflusssteuerelementen verbunden, welche zweckmäßigerweise jeweils für ein Kalibriergas vorgesehen sind. Beispielsweise kann eine Mischung aus 5 ppm $CO_2$ in Stickstoff als ein erstes Kalibriergas vorgesehen sein und eine Mischung aus 5 ppm CO in Stickstoff als ein zweites Kalibriergas.

Weitere Vorteile und Ausgestaltungen der Erfindung ergeben sich aus der Beschreibung und der beiliegenden Zeichnung.

Es versteht sich, dass die vorstehend genannten und die nachfolgend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand eines Ausführungsbeispiels in der Zeichnung schematisch dargestellt und wird im Folgenden unter Bezugnahme auf die Zeichnung ausführlich beschrieben.

Figurenbeschreibung

Figur 1 zeigt schematisch ein Gasverdünnungssystem nach dem Stand der Technik.

Figur 2 zeigt schematisch eine bevorzugte Ausgestaltung eines erfindungsgemäßen Gasverdünnungssystems.

Figur 3 zeigt schematisch eine weitere bevorzugte Ausgestaltung eines erfindungsgemäßen Gasverdünnungssystems.

Figur 4 zeigt schematisch einen zeitlichen Verlauf einer Durchflussmenge und eines Drucks, welche an einer bevorzugten Ausgestaltung eines erfindungsgemäßen Gasverdünnungssystems erfasst werden können.

Ausführungsform(en) der Erfindung

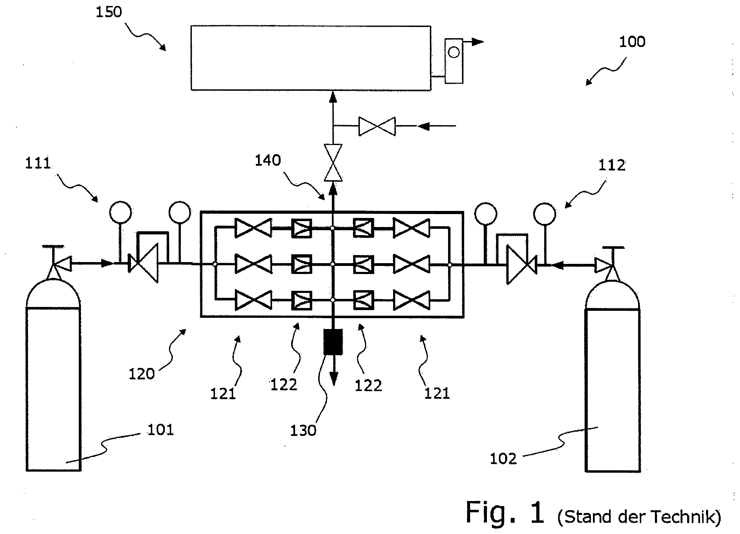

In Figur 1 ist ein Gasverdünnungssystem nach dem Stand der Technik schematisch dargestellt und mit 100 bezeichnet.

Dieses herkömmliche Gasverdünnungssystem 100 umfasst einen ersten Druckgasbehälter 101 und einen zweiten Druckgasbehälter 102. In dem ersten Druckgasbehälter 101 ist beispielsweise ein Ausgleichsgas, z.B. Stickstoff enthalten, in dem zweiten Druckgasbehälter 102 ein Kalibriergas, beispielsweise eine Mischung aus 5 ppm $CO_2$ in Stickstoff.

Für den ersten und den zweiten Druckgasbehälter 101 bzw. 102 ist jeweils ein Druckregler 111 bzw. 112 vorgesehen. Eine Verdünnungseinheit 120 ist vorgesehen, um eine bestimmte Menge an Ausgleichsgas aus dem ersten Behälter 101 mit einer bestimmten Menge an Kalibriergas aus dem zweiten Behälter 102 miteinander zu vermischen und ein verdünntes Gasgemisch zu erzeugen.

Diese Verdünnungseinheit 120 umfasst eine Vielzahl von Ventilen 121 und Messblenden 122. Gemäß diesem Beispiel sind jeweils sechs Ventile 121 und sechs Messblenden 122 vorgesehen, um das verdünnte Gasgemisch in einem gewünschten Verdünnungsverhältnis erzeugen zu können.

Ein Regler 130 ist zur Aufrechterhaltung einer geforderten Durchflussmenge an verdünntem Gasgemisch und zum Ablassen von nicht benötigtem verdünntem Gasgemisch vorgesehen.

Das erzeugte Gasgemisch wird an einem Ausgang 140 bereitgestellt. Beispielsweise ist an dem Ausgang 140 ein Gasanalysenmessgerät 150 angeschlossen. Das erzeugte verdünnte Gasgemisch kann beispielsweise zur Kalibrierung des Gasanalysenmessgeräts 150 verwendet werden.

Ein derartiges herkömmliches Gasverdünnungssystem 100 ist mit vielen Nachteilen behaftet. Durch die Verwendung einer Vielzahl von Ventilen 121 und Messblenden 122 ist das Gasverdünnungssystem 100 sehr sperrig und mit einem hohen Platzbedarf verbunden sowie sehr kostenintensiv in Betrieb und Herstellung. Weiterhin ist ein derartiges herkömmliches Gasverdünnungssystem 100 durch die hohe Anzahl von Bauteilen kompliziert zu handhaben.

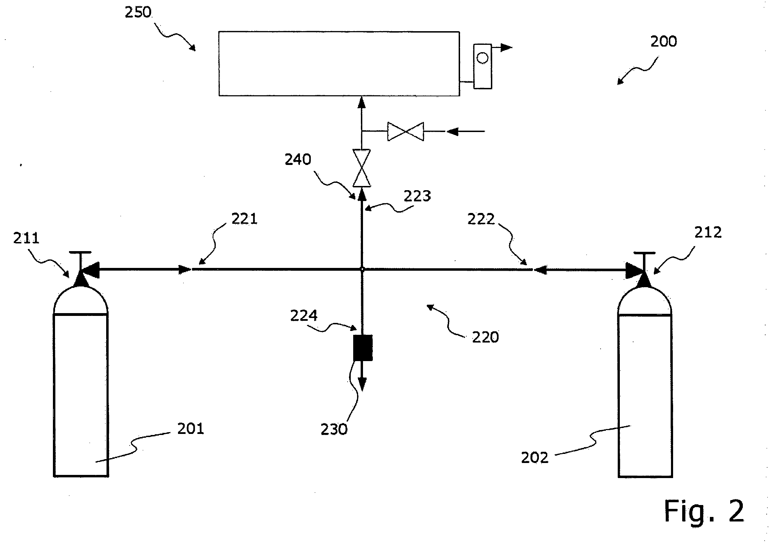

Diese Nachteile können durch eine bevorzugte Ausgestaltung eines erfindungsgemäßen Gasverdünnungssystems behoben werden, welches in Figur 2 schematisch dargestellt und mit 200 bezeichnet ist.

Das Gasverdünnungssystem 200 umfasst in diesem Beispiel ein erstes Gasdurchflusssteuerelement 211 und ein zweites Gasdurchflusssteuerelement 212. Jedes der Gasdurchflusssteuerelemente 211 und 212 umfasst jeweils einen integrierten Gasdruckregler und eine integrierte Messblende, die beispielsweise als integrierter Durchflussregler ausgebildet sein kann.

Insbesondere sind die Gasdurchflusssteuerelemente 211 und 212 jeweils fest mit einem Gasbehälter bzw. Druckgasbehälter 201 und 202 verbunden. Ein erster Gasbehälter 201 und das erste Gasdurchflusssteuerelement 211 sowie ein zweiter Gasbehälter 202 und das zweite Gasdurchflusssteuerelement 212 bilden jeweils eine gemeinsame bauliche Einheit. Die Gasdurchflusssteuerelemente 211 und 212 sind zweckmäßigerweise jeweils als Aufsatz nach Art eines Ventils ausgebildet.

In dem ersten Gasbehälter 201 kann beispielsweise ein Ausgleichsgas wie Stickstoff enthalten sein, in dem zweiten Gasbehälter 202 ein Kalibriergas, beispielsweise eine Mischung aus 5 ppm $CO_2$ in Stickstoff.

Eine Gaszusammenführung 220 ist mit den Gasdurchflusssteuerelementen 211 und 212 verbunden und dazu vorgesehen, um aus den Gasbehältern 201 und 202 entnommene Gase zusammenzuführen und zu einem verdünnten Gasgemisch zu vermischen.

Über die Gasdurchflusssteuerelemente 211 und 212 kann Gas aus den jeweiligen Gasbehältern 201 bzw. 202 entnommen werden. Durch Einstellen der Durchmesser der Messblenden kann ein Durchfluss des aus den jeweiligen Gasbehälter 201 bzw. 202 entnommenen Gases eingestellt werden. Durch Einstellen dieser Durchflüsse kann das Verdünnungsverhältnis des erzeugten verdünnten Gasgemischs eingestellt werden.

Die integrierten Messblenden der Gasdurchflusssteuerelemente 211 und 212 sind insbesondere jeweils als Messblenden für kritische Strömungen ausgebildet, um jeweils eine kritische Strömung durch das Gasdurchflusssteuerelement 211 bzw. 212 zu erzeugen. Die Durchflüsse dieser kritischen Strömungen sind insbesondere jeweils nur abhängig von der Temperatur und dem Druck bzw. der Dichte stromaufwärts der jeweiligen Messblende und können daher zweckmäßigerweise mittels des jeweiligen Gasdruckreglers stromaufwärts der jeweiligen Messblende auf einfache und unkomplizierte Weise eingestellt bzw. geregelt werden.

Die Gaszusammenführung 220 ist in diesem Beispiel als ein Kreuzstück ausgebildet. Ein erster Anschluss 221 des Kreuzstücks 220 ist mit dem ersten Gasdurchflusssteuerelement 211 verbunden und ein zweiter Anschluss 222 des Kreuzstücks 220 ist mit dem zweiten Gasdurchflusssteuerelement 212 verbunden.

Ein dritter Anschluss 223 des Kreuzstücks 220 ist mit einem Ausgang 240 des Gasverdünnungssystems 200 verbunden, an welchem das erzeugte Gasgemisch bereitgestellt wird. Beispielsweise kann an dem Ausgang 240 analog zu Figur 1 ein Gasanalysenmessgerät 250 angeschlossen, welche mittels des erzeugten verdünnten Gasgemischs kalibriert wird.

Ein vierter Anschluss 224 des Kreuzstücks 220 ist mit einem Übermengenregler 230 verbunden. Der Übermengenregler 230 ist in diesem Beispiel als ein Nadelventil oder Rückdruckregler ausgebildet, Gemäß der Erfindung ist der Übermenaenrealer 230 dazu vorgesehen, um eine geforderte Durchflussmenge an verdünntem Gasgemisch aufrecht zu erhalten und um nicht benötigtes verdünntes Gasgemisch abzulassen.

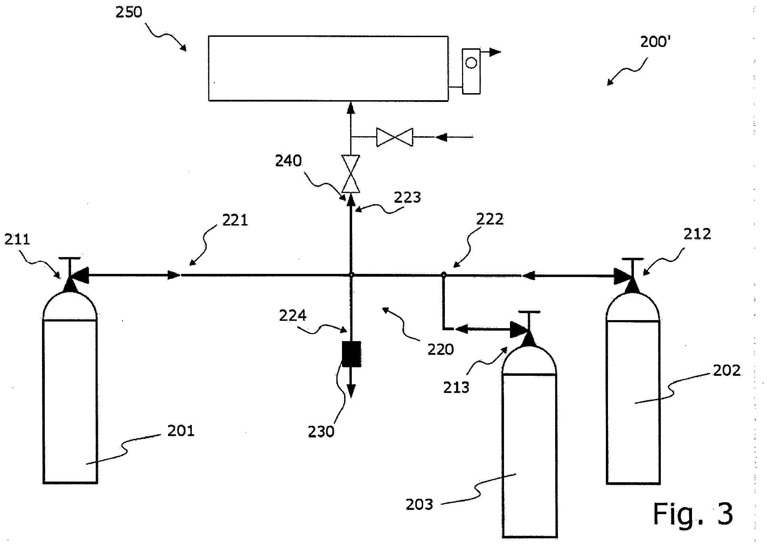

In Figur 3 ist eine weitere bevorzugte Ausgestaltung eines erfindungsgemäßen Gasverdünnungssystems schematisch dargestellt und mit 200' bezeichnet. Identische Bezugzeichen in den Figuren 2 und 3 beschreiben gleiche oder baugleiche Elemente. Im Gegensatz zu dem Gasverdünnungssystem 200 gemäß Figur 2 weist das Gasverdünnungssystem 200' ein drittes Gasdurchflusssteuerelement 213 und einen dritten Gasbehälter 203 auf, die zusammen ebenfalls eine gemeinsame bauliche Einheit bilden. Das Gasdurchflusssteuerelement 213 ist analog zu den Gasdurchflusssteuerelementen 211 und 212 als Flaschenventil mit integriertem Gasdruckregler und integrierter Messblende ausgebildet. Beispielsweise ist eine Mischung aus 5 ppm CO in Stickstoff als ein weiteres Kalibriergas in dem dritten Gasbehälter 203 enthalten. Der zweite Anschluss 222 des Kreuzstücks 220 ist in diesem Beispiel sowohl mit dem zweiten Gasdurchflusssteuerelement 212 als auch mit dem dritten Gasdurchflusssteuerelement 213 verbunden.

Um die Durchflüsse der aus den Gasbehältern entnommenen Gase bestmöglich regeln zu können, ist insbesondere eine Genauigkeit des Durchflusses durch das jeweilige Gasdurchflusssteuerelement bei einem bestimmten Druck bekannt. Insbesondere sind zu diesem Zweck Kurven der Durchflussmenge gegen die Zeit bzw. des Drucks gegen die Zeit der jeweiligen Gasdurchflusssteuerelemente bekannt.

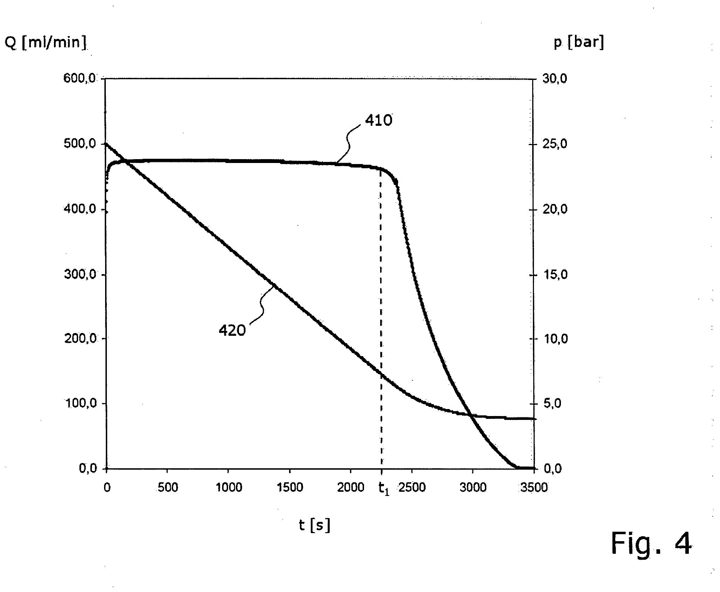

In Figur 4 ist ein entsprechendes Diagramm schematisch dargestellt, wobei Kurve 410 den zeitlichen Verlauf des Durchflusses bzw. der Durchflussmenge Q in der Einheit [ml/min] beschreibt und Kurve 420 den zeitlichen Verlauf des Drucks in der Einheit [bar]. Bis zu einem Zeitpunkt $t_1$ kann die Durchflussmenge Q konstant bzw. im Wesentlichen konstant aufrechterhalten werden.

Im Folgenden wird beispielhaft beschrieben, wie die Konzentration bzw. das Verdünnungsverhältnis des am Ausgang 240 des Gasverdünnungssystems 200 gemäß Figur 2 bereitgestellten verdünnten Gasgemischs berechnet werden kann.

$x_{outlet}$ bezeichnet die Konzentration bzw. das Verdünnungsverhältnis des am Ausgang 240 bereitgestellten verdünnten Gasgemischs im ppb-Bereich. $x_{inlet}$ bezeichnet die Konzentration bzw. das Verdünnungsverhältnis des Kalibriergases im zweiten Gasbehälter 202.

$Q_{pure}$ bezeichnet dabei die Durchflussmenge des Ausgleichsgases, das über das erste Gasdurchflusssteuerelement 211 entnommen wird, $Q_{cal}$ bezeichnet die Durchflussmenge des Kalibriergases, welches über das zweite Gasdurchflusssteuerelement 212 entnommen wird.

Die Konzentration $x_{outlet}$ berechnet sich insbesondere wie folgt:

$$x_{outlet} = x_{inlet} \frac{Q_{cal}}{Q_{cal} + Q_{pure}}$$

Eine Messunsicherheit bzw. Ungenauigkeit $u(x_{outlet})$ dieser Konzentration in Abhängigkeit von der Messunsicherheit $u(x_{inlet})$ der Konzentration des Kalibriergases, von der Messunsicherheit $u(Q_{cal})$ der Durchflussmenge des Kalibriergases sowie von der Messunsicherheit $u(Q_{pure})$ des Ausgleichsgases berechnet sich insbesondere wie folgt:

$$u\left(x_{outlet}\right)^2 = \left(\frac{Q_{cal}}{Q_{cal}+Q_{pure}} u\left(x_{inlet}\right)\right)^2 +$$
$$+ \left(\left(\frac{x_{inlet}}{Q_{cal}+Q_{pure}} - \frac{Q_{cal} x_{inlet}}{\left(Q_{cal}+Q_{pure}\right)^2}\right) u\left(Q_{cal}\right)\right)^2 +$$
$$+ \left(\frac{Q_{cal} x_{inlet}}{\left(Q_{cal}+Q_{pure}\right)^2} u\left(Q_{pure}\right)\right)^2$$

Bezugszeichenliste

| | |
|---|---|
| 100 | Gasverdünnungssystem nach Stand der Technik |
| 101 | erster Druckbehälter |
| 102 | zweiter Druckbehälter |
| 111 | erster Druckregler |
| 112 | zweiter Druckregler |
| 120 | Verdünnungseinheit |
| 121 | Ventile |
| 122 | Messblenden |
| 130 | Nadelventil |
| 140 | Ausgang |
| 150 | Gasanalysenmessgerät |
| | |
| 200 | Gasverdünnungssystem |
| 200' | Gasverdünnungssystem |
| 201 | erster Druckbehälter |
| 202 | zweiter Druckbehälter |
| 203 | dritter Druckbehälter |
| 211 | erstes Gasdurchflusssteuerelement, Flaschenventil |
| 212 | zweites Gasdurchflusssteuerelement, Flaschenventil |
| 213 | drittes Gasdurchflusssteuerelement, Flaschenventil |
| 220 | Gaszusammenführung, Kreuzstück |
| 221 | erster Anschluss des Kreuzstücks |
| 222 | zweiter Anschluss des Kreuzstücks |
| 223 | dritter Anschluss des Kreuzstücks |
| 224 | vierter Anschluss des Kreuzstücks |
| 230 | Übermengenregler (z.B. Nadelventil oder Rückdruckregler) |
| 240 | Ausgang |
| 250 | Gasanalysenmessgerät |
| | |
| 410 | zeitlicher Verlauf der Durchflussmenge Q |
| 420 | zeitlicher Verlauf des Drucks |

Patentansprüche

1. Gasverdünnungssystem (200, 200')

mit wenigstens zwei Gasdurchflusssteuerelementen (211, 212, 213), und wenigstens zwei Gasbehältern (201, 202, 203),
wobei jedes der wenigstens zwei Gasdurchflusssteuerelemente (211, 212, 213) jeweils eine integrierte Messblende aufweist,
wobei jedes der wenigstens zwei Gasdurchflusssteuerelemente (211, 212, 213) jeweils als Aufsatz nach Art eines Ventils derart ausgebildet ist, dass jedes der wenigstens zwei Gasdurchflusssteuerelemente (211, 212, 213) jeweils direkt auf einen der jeweiligen Gasbehälter (201, 202, 203) aufgesetzt und mit diesem jeweiligen Gasbehälter (201, 202, 203) lösbar oder fest verbunden ist,
wobei das Gasverdünnungssystem (200, 200') dazu eingerichtet ist, über die wenigstens zwei Gasdurchflusssteuerelemente (211, 212, 213) jeweils Gas mit einem vorgegebenen Durchfluss aus dem jeweiligen verbundenen Gasbehälter (201, 202, 203) zu entnehmen, die entnommenen Gase in einem vorgegebenen Verdünnungsverhältnis zu einem verdünnten Gasgemisch zu vermischen und das verdünnte Gasgemisch an einem Ausgang (240) bereitzustellen,
wobei das vorgegebene Verdünnungsverhältnis des verdünnten Gasgemischs über Blendendurchmesser der integrierten Messblenden der wenigstens zwei Gasdurchflusssteuerelemente (211, 212, 213) einstellbar ist und **dadurch gekennzeichnet, dass** das Gasverdünnungssystem einen Übermengenregler (230) aufweist, welcher dazu vorgesehen ist, eine geforderte Durchflussmenge an verdünntem Gasgemisch aufrecht zu erhalten und nicht benötigtes verdünntes Gasgemisch abzulassen.

2. Gasverdünnungssystem (200, 200') nach einem der vorstehenden Ansprüche, wobei die wenigstens zwei Gasdurchflusssteuerelemente (211, 212, 213) jeweils weiterhin einen integrierten Gasdruckregler umfassen.

3. Gasverdünnungssystem (200, 200') nach einem der vorstehenden Ansprüche, wobei die wenigstens zwei Gasdurchflusssteuerelemente (211, 212, 213) mit dem jeweiligen verbundenen Gasbehälter (201, 202, 203) jeweils eine gemeinsame bauliche Einheit bilden.

4. Gasverdünnungssystem (200, 200') nach Anspruch 1, wobei der, Übermengenregler (230), ein Nadelventil und/oder ein Rückdruckregler ist.

5. Gasverdünnungssystem (200, 200') nach einem der vorstehenden Ansprüche, weiterhin aufweisend eine Gaszusammenführung (240), insbesondere als ein Kreuzstück (220) von Gasleitungen ausgebildet, welche mit den wenigstens zwei Gasdurchflusssteuerelementen (211, 212, 213) verbunden ist.

6. Gasverdünnungssystem (200, 200') nach Anspruch 65, wobei die Gaszusammenführung (240) als ein Kreuzstück (220) ausgebildet ist, und

ein erster Anschluss (221) des Kreuzstücks (220) mit einer ersten Anzahl der wenigstens zwei Gasdurchflusssteuerelemente (211) verbunden ist,
ein zweiter Anschluss (222) des Kreuzstücks (220) mit einer zweiten Anzahl der wenigstens zwei Gasdurchflusssteuerelemente (212, 213) verbunden ist,
ein dritter Anschluss (223) des Kreuzstücks (220) mit dem Ausgang (240) verbunden ist und ein vierter Anschluss (224) des Kreuzstücks (220) mit dem Übermengenregler (230) verbunden ist.

## Claims

1. Gas dilution system (200, 200')

having at least two gas flow control elements (211, 212, 213), and at least two gas tanks (201, 202, 203),
wherein each of the at least two gas flow control elements (211, 212, 213) each has an integrated measuring orifice,
wherein each of the at least two gas flow control elements (211, 212, 213) is each designed as an attachment in the manner of a valve such that each of the at least two gas flow control elements (211, 212, 213) is each placed directly on one of the respective gas tanks (201, 202, 203) and is detachedly or fixedly connected to said respective gas tank (201, 202, 203),
wherein the gas dilution system (200, 200') is configured to extract gas having a predetermined flow from the respective connected gas tank (201, 202, 203) via the at least two gas flow control elements (211, 212, 213) in each case, to mix the extracted gases in a predetermined dilution ratio into a diluted gas mixture, and to provide the diluted gas mixture at an outlet (240), wherein the predetermined dilution ratio of the diluted gas mixture may be adjusted via orifice diameters of the integrated measuring orifices of the at least two gas flow control elements (211, 212, 213) and **characterized in that** the gas dilution system has a surplus flow regulator (230) which is provided to maintain a required flow volume of diluted gas mixture and to discharge diluted gas mixture that is not required.

2. Gas dilution system (200, 200') according to any one of the preceding claims, wherein the at least two gas flow control elements (211, 212, 213) each further comprises an integrated gas pressure regulator.

3. Gas dilution system (200, 200') according to any one of the preceding claims, wherein the at least two gas flow control elements (211, 212, 213) each form a common structural unit with the respective connected gas tank (201, 202, 203).

4. Gas dilution system (200, 200') according to claim 1, wherein the surplus flow regulator (230) is a needle valve and/or a back pressure regulator.

5. Gas dilution system (200, 200') according to any one of the preceding claims, further comprising a gas junction (240), which is in particular designed as a crosspiece (220) of gas lines and is connected to the at least two gas flow control elements (211, 212, 213).

6. Gas dilution system (200, 200') according to claim 5, wherein the gas junction (240) is designed as a crosspiece (220), and

> a first connection (221) of the crosspiece (220) is connected to a first number of the at least two gas flow control elements (211),
> a second connection (222) of the crosspiece (220) is connected to a second number of the at least two gas flow control elements (212, 213), a third connection (223) of the crosspiece (220) is connected to the outlet (240) and a fourth connection (224) of the crosspiece (220) is connected to the surplus flow regulator (230).

## Revendications

1. Système de dilution de gaz (200, 200')

> présentant au moins deux éléments de commande d'écoulement de gaz (211, 212, 213) et au moins deux récipients à gaz (201, 202, 203), chaque élément desdits au moins deux éléments de commande d'écoulement de gaz (211, 212, 213) présentant à chaque fois une plaque à diaphragme intégrée,
> chaque élément desdits au moins deux éléments de commande d'écoulement de gaz (211, 212, 213) étant conçu à chaque fois comme embout de la manière d'une soupape, de manière telle que chaque élément desdits au moins deux éléments de commande d'écoulement de gaz (211, 212, 213) est placé à chaque fois directement sur l'un des récipients à gaz respectifs (201, 202, 203) et est relié de manière amovible ou fixe à ce récipient à gaz respectif (201, 202, 203),
le système de dilution de gaz (200, 200') étant conçu pour prélever, par l'intermédiaire desdits au moins deux éléments de commande d'écoulement de gaz (211, 212, 213), à chaque fois un gaz à un écoulement défini à partir du récipient à gaz respectif relié (201, 202, 203), pour mélanger les gaz prélevés dans un rapport de dilution défini en un mélange gazeux dilué et pour mettre à disposition le mélange gazeux dilué au niveau d'une sortie (240),
le rapport de dilution défini du mélange gazeux dilué étant réglable par l'intermédiaire des diamètres des plaques à diaphragme intégrées desdits au moins deux éléments de commande d'écoulement de gaz (211, 212, 213) et **caractérisé en ce que** le système de dilution de gaz présente un régulateur de quantité excédentaire (230) qui est conçu pour maintenir un débit requis de mélange gazeux dilué et pour évacuer du mélange gazeux dilué non nécessaire.

2. Système de dilution de gaz (200, 200') selon l'une quelconque des revendications précédentes, lesdits au moins deux éléments de commande d'écoulement de gaz (211, 212, 213) comprenant à chaque fois en outre un régulateur intégré de pression gazeuse.

3. Système de dilution de gaz (200, 200') selon l'une quelconque des revendications précédentes, lesdits au moins deux éléments de commande d'écoulement de gaz (211, 212, 213) formant à chaque fois une unité modulaire commune avec le récipient à gaz respectif relié (201, 202, 203).

4. Système de dilution de gaz (200, 200') selon la revendication 1, le régulateur de quantité excédentaire (230) étant une soupape à aiguille et/ou un régulateur de pression en retour.

5. Système de dilution de gaz (200, 200') selon l'une quelconque des revendications précédentes, présentant en outre un rassemblement des gaz (204), en particulier conçu comme un croisement (220) de conduites à gaz, qui est relié auxdits au moins deux éléments de commande d'écoulement de gaz (211, 212, 213).

6. Système de dilution de gaz (200, 200') selon la revendication 5, le regroupement de gaz (240) étant conçu comme un croisement (220) et

> un premier raccord (221) du croisement (220) étant relié à un premier nombre desdits au moins deux éléments de commande d'écoulement de gaz (211),
> un deuxième raccord (222) du croisement (220) étant relié à un deuxième nombre desdits au moins deux éléments de commande d'écoulement de gaz (212, 213),
un troisième raccord (223) du croisement (220) étant relié à la sortie (240) et un quatrième raccord (224) du croisement (220) étant relié au régulateur de quantité excédentaire (230).

Fig. 1 (Stand der Technik)

Fig. 2

Fig. 3

Fig. 4

**IN DER BESCHREIBUNG AUFGEFÜHRTE DOKUMENTE**

**In der Beschreibung aufgeführte Patentdokumente**

- DE 194166
- WO 2007050400 A1
- EP 0503996 A1